**Europäisches Patentamt**

**European Patent Office**

**Office européen des brevets**

(11) Veröffentlichungsnummer: **0 143 185 B1**

(12) **EUROPÄISCHE PATENTSCHRIFT**

(45) Veröffentlichungstag der Patentschrift: **24.06.87**

(21) Anmeldenummer: **84109795.9**

(22) Anmeldetag: **17.08.84**

(51) Int. Cl.⁴: **A 61 F 9/00**

(54) **Ophthalmologisches Kombinationsgerät für Diagnose und Therapie.**

(30) Priorität: **01.09.83 DE 3331586**

(43) Veröffentlichungstag der Anmeldung:
**05.06.85 Patentblatt 85/23**

(45) Bekanntmachung des Hinweises auf die Patenterteilung:
**24.06.87 Patentblatt 87/26**

(84) Benannte Vertragsstaaten:
**CH DE FR GB IT LI**

(56) Entgegenhaltungen:
GB - A - 2 074 343
US - A - 3 481 672
US - A - 3 491 240

LASER FOCUS, Band 19, Nr. 1, Januar 1983, Seiten
38,39; "Medical applications"
SOVIET JOURNAL OF QUANTUM ELECTRONICS, Band
11, Nr. 12, Dezember 1981, Seiten 1606-1611, American
Institute of Physics, New York, USA; D.H. SLINEY:
"Mechanisms of interaction of laser radiation with
ocular tissues: implications for human exposure limits"
ALTA FREQUENZA, Band 41, Nr. 10, Oktober 1972,
Seiten 771-779; D. ROSENBERGER: "Laser sensors for
industry"

(73) Patentinhaber: **Firma Carl Zeiss, D-7920 Heidenheim (Brenz) (DE)**
(84) Benannte Vertragsstaaten: **CH DE FR GB IT LI**

(73) Patentinhaber: **CARL-ZEISS-STIFTUNG trading as CARL ZEISS, D-7920 Heidenheim (Brenz) (DE)**
(84) Benannte Vertragsstaaten: **GB**

(72) Erfinder: **Müller, Ortwin, Dipl.-Phys., Kolpingstrasse 34, D-7080 Aalen (DE)**
Erfinder: **Schulz, Kurt, Mozartweg 20, D-7082 Oberkochen (DE)**
Erfinder: **Vogel, Albrecht, Hainbuchenweg 14, D-7082 Oberkochen (DE)**
Erfinder: **Hanemann, Gerhard, Jenaer Strasse 4, D-7082 Oberkochen (DE)**
Erfinder: **Müller, Gerhard, Prof. Dr., Philipp-Funk-Strasse 112, D-7080 Aalen (DE)**
Erfinder: **Kürbitz, Gunther, Dr., Helfensteinstrasse 5, D-7923 Königsbronn-Zang (DE)**
Erfinder: **Güttner, Arnold, Dr., Holunderweg 6, D-7923 Königsbronn (DE)**

**Beschreibung**

Die Erfindung betrifft ein ophthalmologisches Kombinationsgerät, bestehend aus einem stereoskopischen Beobachtungsmikroskop und einem an die Stelle seines Hauptobjektivs einsetzbaren Zusatzgerät.

Ein ophthalmologisches Kombinationsgerät ist beispielsweise aus der DE-C-2 614 273 bekannt. Bei diesem bekannten Kombinationsgerät handelt es sich um ein Diagnosegerät, bei dem eine Spaltlampe, die nach dem Prinzip der binokularen Fernrohrlupe aufgebaut ist, nach Entfernen des Haupt-Objektivs des stereoskopischen Beobachtungsmikroskops durch Einfügen eines weiteren optischen Teiles in ein Ophthalmometer verwandelt werden kann.

Ausserdem sind Operationsmikroskope bekannt, die für Therapiezwecke mit einem Lasersystem kombinierbar sind. Derartige Geräte sind beispielsweise in der US-A-3 769 693 und in der USA-3 096 767 beschrieben. In diesen beiden bekannten Geräten wird die Therapie-Laserstrahlung ausserhalb des Beobachtungsgerätes bzw. des Operationsmikroskops geführt.

Aus der Druckschrift LASER FOCUS, Band 19, Nr. 1, Januar 1983, Seiten 38, 39 ist ein Spaltlampengerät bekannt, in dessen Strahlengang für Therapiezwecke die Strahlung eines neben der Spaltlampe angeordneten gross bauenden Lasers eingeführt wird.

Für die Kombination eines Lasersystems für Therapiezwecke mit einem stereoskopischen Beobachtungsmikroskop nach der DE-C-2 614 273 ist ein derartig gross bauender Laser ungeeignet. Dafür ist ein klein bauender Laser erforderlich, dessen Strahlung durch die Beobachtungsoptik des binokularen Beobachtungsmikroskops geführt werden kann. Es hat sich überraschend gezeigt, dass sich ein aus Entfernungsmesssystemen bekannter Neodym-YAG Impuls-Laser (Nd: YAG-Laser) mit passivem Güteschalter (Q-Switch) für diesen Zweck besonders gut eignet, weil er die Eigenschaft hat, dass er bei Erhöhung der Pumplichtenergie in einer Impulsfolge nicht einen einzelnen Impuls, sondern eine Impulskette von mehreren, z.B. bis zu fünf Einzelimpulsen aussendet, wobei der zeitliche Abstand der Impulse bei 10-20 µ sec und die Energie des Einzelimpulses bei ca. 15-20 mJ liegt. Beim bisherigen Einsatzgebiet dieses bekannten Lasers ist diese Eigenschaft unerwünscht und wird durch entsprechende Wahl der Betriebsbedingungen vermieden. Bei der Katarakttherapie am Auge hat sich diese Eigenschaft aber als nützlich herausgestellt. Derartige Laserimpulse bewirken bei Fokussierung eine spontane Ionisierung der Materie und zerstören dadurch die Proteinfibrillen, die eine Linsen oder Glaskörpertrübung verursachen. Es hat sich herausgestellt, dass es für gezielte Führung des therapeutischen Effektes auf eben diesen spontanen Materiezusammenbruch und die damit verbundene mechanische Schockwelle ankommt, die zum Cracken der Proteinfibrillen führt. Der genannte Nd: YAG-Laser eignet sich wegen seiner kleinen Ausmessungen für den angestrebten Therapiezweck optimal, wenn er mit genügend grosser Pumplichtenergie betrieben wird. Ebenso kann der genannte Laser auch mit Vorteil zur Perforation der Linsenmembran oder der Iris selbst eingesetzt werden.

Die Erfindung hat sich deshalb die Aufgabe gestellt, ein handliches Kombinationsgerät für Diagnose und Therapiezwecke nach dem aus der DE-C-2 614 273 bekannten Kombinationsprinzip zu schaffen.

Diese Aufgabe wird erfindungsgemäss dadurch gelöst, dass als Zusatzgerät die Kombination eines Lasersystems aus einem im unsichtbaren Bereich gepulst emittierenden Laser, der in einem Pumpvorgang mehrere Einzelimpulse mit einer Dauer von je ca. 5 n sec, einem zeitlichen Abstand von je ca. 10 µ sec und einer Energie von ca. 15-20 mJ erzeugt, mit einem im sichtbaren Bereich kontinuierlich emittierenden Lager vorgesehen ist, dessen Querschnitt aus zwei wechselweise ein- und ausschaltbaren Teilbündeln besteht, zwischen denen sich eine lichtfreie Zone befindet, wobei der Schwerstrahl der sichtbaren und der unsichtbaren Laserstrahlung in einer Achse liegen.

Zweckmässigerweise sind im Beobachtungsstrahlengang Konversionsfilter und Laserschutzfilter vorgesehen.

In einem vorteilhaften Ausführungsbeispiel der Erfindung sind die Teilbündel der sichtbaren Laserstrahlung als Segmente eines Kreises ausgebildet, dessen Radius grösser ist als der Radius des Kreises der unsichtbaren Laserstrahlung und werden mittels einer elektrischen Schaltung wechselweise ein- und ausgeschaltet.

Zum Ein- und Ausschalten der Teilbündel kann auch eine Stabblende vorgesehen sein, die über den Strahlquerschnitt hin- und herbewegt wird.

In einem vorteilhaften weiteren Ausführungsbeispiel besteht die Stabblende aus Federdraht, der über einen motorgetriebenen umlaufenden Exzenter hin- und herbewegt wird.

Eine besonders günstige Bauweise ergibt sich, wenn für die mechanisch-optischen Teile des Laser-Therapiezusatzes ein Gehäuse vorgesehen ist, das sich oberhalb der Ebene befindet, die die Mikroskopachsen des Beobachtungssystems enthält und das auf der Benutzerseite eine Bedienungsfrontplatte aufweist.

Die mit der Erfindung erzielten Vorteile bestehen insbesondere darin, dass das verwendete Lasersystem eine äusserst kleine Bauform mit einer leichten Modifizierbarkeit der Pumplichtenergie verbindet. Ausserdem bietet die Erfindung dem Chirurgen eine Entlastung durch eine Fokussierhilfe für den Laser-Therapiestrahl und eine äusserst günstige ergonomische Anordnung der Bedienungselemente. Der Benutzer kann die ihm geläufige Spaltlampe in gewohnter Weise zu diagnostischen Zwecken benutzen und nach der Diagnose mittels des leicht bedienbaren therapeutischen Zusatzgerätes die Behandlung einleiten, wobei die Gestaltung des Lagergehäuses dem Benutzer eine gute Sicht auf den Patienten erlaubt.

Die Therapie mit aufeinanderfolgenden Impulsen hat weiterhin den Vorteil, dass im Plasma, das vom ersten Impuls durch spontane Ionisierung der Materie erzeugt wurde, der zweite Impuls noch effektiver absorbiert wird, wodurch der therapeutische Effekt verbessert wird. Es ergibt sich damit eine leicht und sicher durchführbare Methode, um lokal grössere Läsionen zu setzen. Für kleinere Läsionen kann die Energie mit Graufiltern abgeschwächt werden.

Ein Ausführungsbeispiel der Erfindung ist in der Zeichnung dargestellt und wird im folgenden näher beschrieben. Es zeigen:

Fig. 1 eine perspektivische Darstellung des erfindungsgemässen Kombinationsgerätes;

Fig. 2a das mechanisch-optische Prinzip des Kombinationsgerätes in einer Schnittdarstellung, welche die Hauptgeräteachse enthält;

Fig. 2b einen Teilschnitt mit Darstellung der Strahlengänge durch das Hauptfokussierobjektiv;

Fig. 3 das mechanisch-optische Prinzip des Kombinationsgerätes in einer Schnittdarstellung, die zur Abbildung nach Fig. 2a senkrecht steht;

Fig. 4a und 4b die Querschnittsform der Laserstrahlbündel;

Fig. 5 einen Schaltmechanismus für die Sektorbündel des im sichtbaren Bereich emittierenden Lasers.

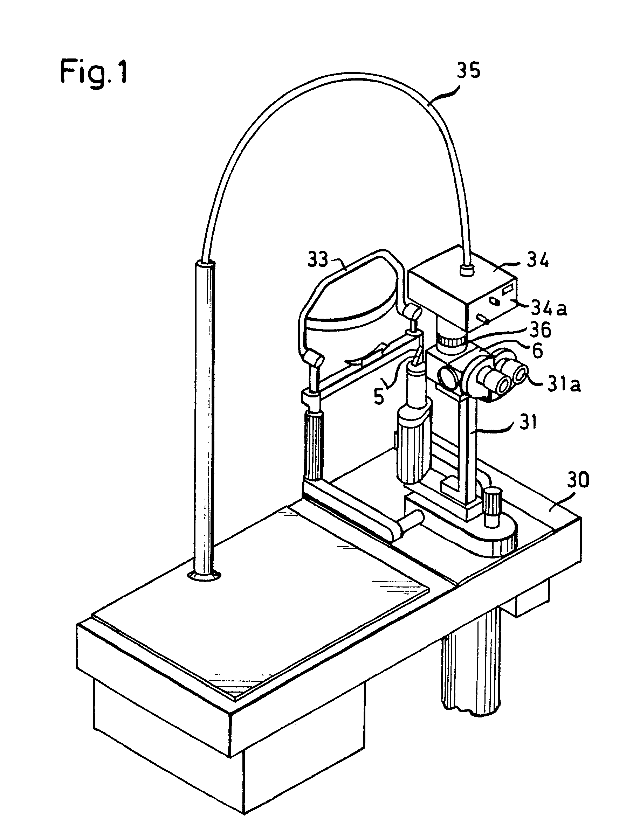

In der perspektivischen Darstellung der Fig. 1 ist mit (30) die Arbeitsplatte bezeichnet, auf der das Kombinationsgerät befestigt ist. Der Träger des stereoskopischen Beobachtungsmikroskops einer Spaltlampe trägt die Bezeichnung (31), die dazu gehörende Spaltbeleuchtung die Bezeichnung (5). Mit (33) ist eine Kopfstütze für den Patienten bezeichnet. Das Lasersystem ist in einem Gehäuse (34) untergebracht, das auf einer Säule über dem Beobachtungsmikroskop (31) steht. Oberhalb des binokularen Einblicks (31a) befindet sich die Bedienungsfrontplatte (34a) des Lagergehäuses. Die Energieversorgung wird aus dem Arbeitstisch (30) über ein Kabel (35) zum Laser (34) gewährleistet.

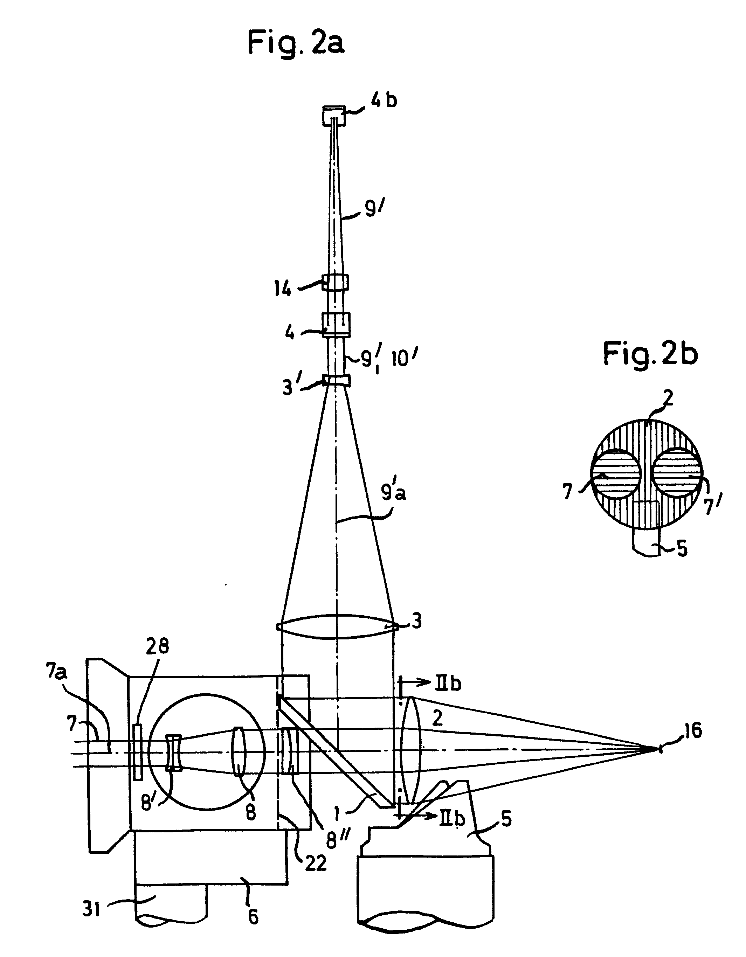

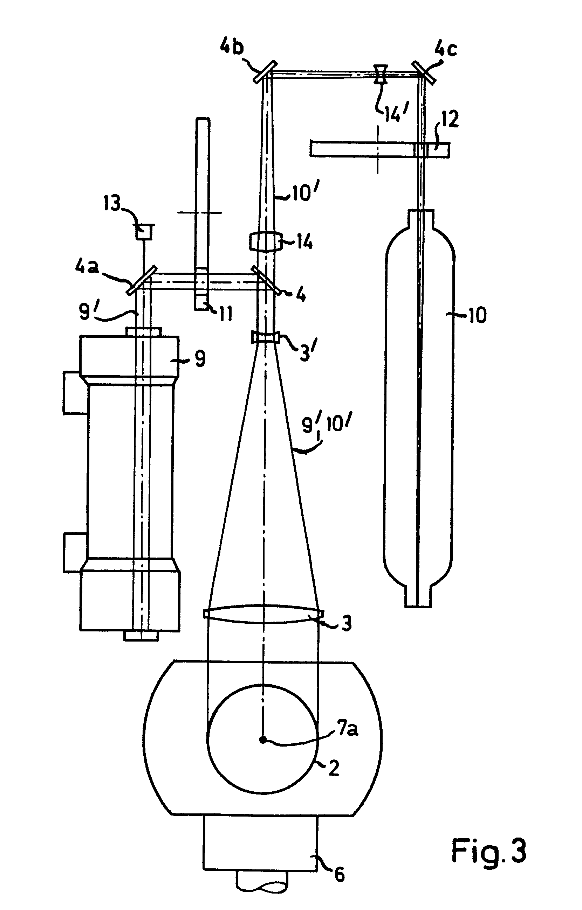

In der Abbildung der Fig. 2a ist der Träger des stereoskopischen Beobachtungsmikroskops der Spaltlampe mit (31) und das Optik-Gehäuseteil mit (6) bezeichnet. Im Beobachtungsstrahlengang (7) ist der bekannte Galilei-Vergrösserungswechsler (8, 8') mit einem Vorsatzsystem (8'') angeordnet. Mit (22) ist die gestrichelt eingezeichnete Trennstelle bezeichnet, auf deren rechter Seite sich normalerweise das Haupt-Objektiv des stereoskopischen Mikroskops befindet, das im Falle der Kombination mit einem Lasersystem entfernt ist. Die Kennziffer (5) bezeichnet die Spaltbeleuchtung, die in der Abbildung der Fig. 2b in Mittenstellung gezeigt ist. Der Einkoppelteil des Laser-Therapiegerätes besteht aus der Teilerplatte (1) und dem Hauptfokussierobjektiv (2). Die Schwerstrahlen (7a) des Beobachtungsstrahlenganges und die Schwerstrahlen (9'a) des Laser-Therapiegerätes fallen zusammen. Für die Laser-Therapiestrahlung wird ein Nd: YAG-Laser und als Markierungs-Laserstrahlgerät ein He-Ne-Laser verwendet. In den Beobachtungsstrahlengängen sind Augenschutzfilter und Konversionsfilter (28) angeordnet. Die beiden Laser sind im Gehäuse (34) untergebracht. In der Darstellung der Fig. 3 ist der Nd: YAG-Laser mit (9) und der He-Ne-Laser mit (10) bezeichnet. Da der Strahl des He-Ne-Lasers einen kleineren Querschnitt hat als der des Nd: YAG-Lasers, wird das von ihm emittierte Strahlenbündel (10') zunächst durch das Fernrohrsystem (14', 14) aufgeweitet, ehe mittels der Teilerplatte (4) eine koaxiale Vereinigung mit dem Therapie-Laserstrahl (9') erfolgt. Durch ein weiteres Fernrohrsystem (3', 3) werden beide Laserstrahlengänge auf den Durchmesser des Objektivs (2) aufgeweitet, wodurch eine grosse Bestrahlungsapertur erzielt wird. Mit (11) und (12) sind abschwächende Filter für die Laserstrahlengänge bezeichnet. (13) ist ein Aufnehmer zur Messung der Energie der Nd: YAG-Laserimpulse. Mit (4a, 4b, 4c) sind Umlenkspiegel bezeichnet.

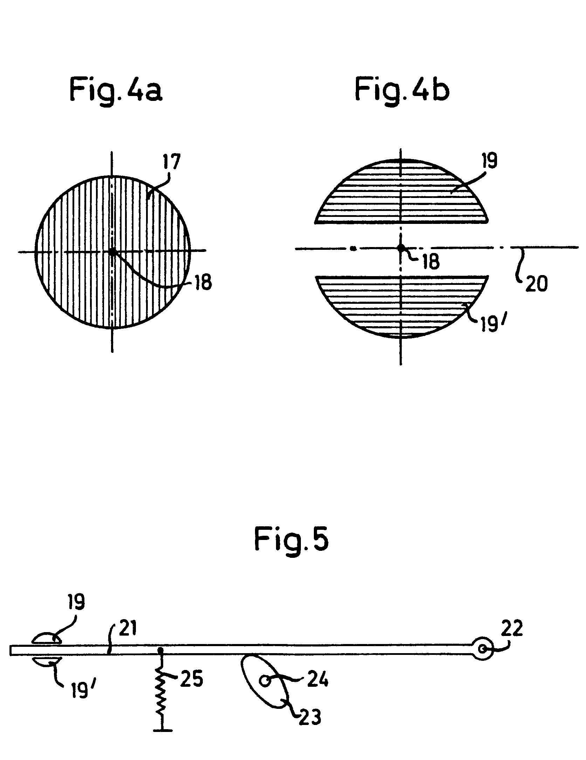

In dem in den Fig. 2a und 3 schematisch dargestellten Ausführungsbeispiel der Erfindung wird die unsichtbare gepulste Neodym-YAG-Strahlung (9') durch das sichtbare He-Ne-Laser-Dauerlicht (10') markiert. Beide Strahlungen sind koaxial und werden im Punkt (16) fokussiert. Durch die feste Zuordnung beider Laser gewinnt der Benutzer die Sicherheit, dass er den Fokus der gefährlichen YAG-Laser-Strahlung stets und unter allen Bedingungen kennt. Zum Zwecke der besseren Fokussierbarkeit sind jedoch die Querschnitte der beiden Laserstrahlbündel unterschiedlich, was anhand der Darstellung der Fig. 4a und 4b näher erläutert wird.

In der Schnittzeichnung der Darstellung der Fig. 4a füllt die Nd: YAG-Strahlung (17) einen Kreisquerschnitt um die Achse (18) aus. Der Strahlquerschnitt des He-Ne-Markierlasers setzt sich dagegen, wie in Fig. 4b dargestellt, aus zwei Segmenten (19 und 19') zusammen, die symmetrisch hinsichtlich der Achse (18) und der Horizontalen (20) liegen. Der Radius des Kreises, aus dem diese Segmente geschnitten sind, ist grösser als der Radius des Kreises der Nd: YAG-Laser-Strahlung. Dadurch wird sichergestellt, dass die Nd: YAG-Strahlung stets innerhalb des Kegels des He-Ne-Laserlichtes emittiert wird. Eine Verschmelzung der beiden Teilbündel (19 und 19') findet lediglich im Fokussierpunkt (16) statt. In jeder anderen Ebene sind die beiden Bündel durch eine lichtfreie Zone getrennt. Diese Ausgestaltung stellt eine ausserordentliche Fokussierhilfe für den Benutzer dar. Sie hat auch zur Folge, dass der Fokus, der auf der Achse (18) entsteht, im optischen Medium kontrastvoll gesehen werden kann, da im ausgeblendeten Bereich zwischen den beiden Teilbündeln kein Streu- und Reflexlicht entstehen kann.

In der Darstellung der Fig. 5 wird ein Mechanismus für eine Fokussierhilfe gezeigt. Sie besteht darin, dass die beiden Teilbündel (19, 19') des He-Ne-Markierlasers (10) abwechselnd aus- und eingeschaltet werden. Wird der Strahlengang von einer Ebene geschnitten, die nicht mit der Fokussierebene identisch ist, so hat der Benutzer den Eindruck des Blinkens der gesehenen Lichtprojektionen. Der Mechanismus der Fokussiereinrichtung besteht in der Hauptsache aus einer Stabblende (21), die senkrecht zur Strahlrichtung über den Querschnitt hin- und herbewegt wird. Dies geschieht mit Hilfe eines motorgetriebenen Exzenters (23), der sich um die Achse (24) dreht. Die Stabblende dreht sich um die Achse (22), und mit (25) ist eine Rückholfeder bezeichnet, die gegen den Exzenter (23) arbeitet. Es ist ersichtlich, dass bei Drehung des Exzenters die Stabblende über die beiden Teilbündel (19 und 19') des Markierlasers hinweggeführt wird, wobei sie dieselben abwechselnd verdeckt. Aufgrund der geringen Divergenz der Laserstrahlung kann der beschriebene Mechanismus vor jedem Aufweitungssystem eingebaut werden. Dies hat den Vorteil, dass er sehr klein gestaltet werden kann. Eine weitere Vereinfachung des beschriebenen Mechanismus besteht dann darin, dass die Stabblende selbst ein Federdraht ist, der am rechten Ende fest eingespannt wird, so dass die Rückholfeder (25) und die Drehachse (22) entfallen. Die Frequenz des Blinkens der Fokussiereinrichtung beträgt etwa ein Hz. Sie lässt sich durch Ändern der Drehzahl des antreibenden Motors variieren. Die dynamische Fokussiereinrichtung mittels der Stabblende lässt sich sehr einfach ausser Betrieb setzen, indem man die Stabblende (21) gegen die Feder (25) so weit nach oben zieht und befestigt, dass der Strahlquerschnitt des Markierlasers ständig frei bleibt.

Da die Modulationstiefe für die Teilbündel 100% beträgt, in der Fokussierebene dagegen höchstens 50%, hat der Benutzer den Eindruck, dass der Fokuspunkt sich zeitkonstant darstellt. Die beschriebene dynamische Fokussiereinrichtung erweist sich insbesondere dann von grossem Vorteil, wenn der Fokus in ein Stystem von spiegelnden Kugelflächen, wie es das Auge darstellt, abgebildet werden soll. Die Spiegelungen können nämlich zu virtuellen Fokussierpunkten führen, die mit dem wahren Fokussierpunkt, der allein der Brechung an den genannten Flächen unterworfen ist, nicht verwechselt werden dürfen. Die Verwechslungsgefahr ist auch deshalb gegeben, weil die Intensität der genannten Reflexe, bezogen auf den Beobachter grösser sein kann, als die Intensität des waren Fokuspunktes, der den Benutzer ja nur durch Streuung und Streureflexion im Medium angezeigt wird. Die beschriebene dynamische Fokussiereinrichtung bewirkt nun, dass alle Reflexpunkte blinken, wobei sie sich dem linken Beobachtungsstrahlengang anders darstellen als dem rechten. Lediglich der wahre Fokuspunkt wird sowohl mit dem rechten als auch mit dem linken Auge in annähernd gleicher Intensität und relativer Ruhe gesehen.

**Patentansprüche**

1. Ophthalmologisches Kombinationsgerät, bestehend aus einem stereoskopischen Beobachtungsmikroskop (31) und einem an die Stelle seines Hauptobjektivs einsetzbaren Zusatzgerät, dadurch gekennzeichnet, dass als Zusatzgerät die Kombination eines Lasersystems aus einem im unsichtbaren Bereich gepulst emittierenden Laser (9), der in einem Pumpvorgang mehrere Einzelimpulse mit einer Dauer von je ca. 5 n sec, einem zeitlichen Abstand voneinander von ca. 10 µ sec und einer Energie von ca. 15 bis 20 mJ erzeugt, mit einem im sichtbaren Bereich kontinuierlich emittierenden Laser (10) vorgesehen ist, dessen Querschnitt aus zwei wechselweise ein- und ausschaltbaren Teilbündeln (19, 19') besteht, zwischen denen sich eine lichtfreie Zone befindet, wobei der Schwerstrahl (9'a) der sichtbaren und der der unsichtbaren Laserstrahlung in einer Achse (18) liegen.

2. Ophthalmologisches Kombinationsgerät nach Anspruch 1, dadurch gekennzeichnet, dass die Teilbündel (19, 19') als Segmente eines Kreises ausgebildet sind, dessen Radius grösser ist als der Radius des Kreises der unsichtbaren Laserstrahlung (9').

3. Ophthalmologisches Kombinationsgerät nach Anspruch 1, dadurch gekennzeichnet, dass im Beobachtungsstrahlengang (7) Konversionsfilter und Laserschutzfilter (28) vorgesehen sind.

4. Ophthalmologisches Kombinationsgerät nach Anspruch 2, dadurch gekennzeichnet, dass eine elektrische Schaltung zur wechselweisen Ein- und Ausschaltung der sichtbaren Laserstrahlung (10') vorgesehen ist.

5. Ophthalmologisches Kombinationsgerät nach Anspruch 4, dadurch gekennzeichnet, dass das Ein- und Ausschalten der Teilbündel (19, 19') durch eine Stabblende (21) geschieht, die über den Strahlquerschnitt hin- und herbewegt wird.

6. Ophthalmologisches Kombinationsgerät nach Anspruch 5, dadurch gekennzeichnet, dass die Stabblende (21) aus Federdraht besteht, der über einen motorgetriebenen umlaufenden Exzenter (23) hin- und herbewegt wird.

7. Ophthalmologisches Kombinationsgerät nach Anspruch 1, dadurch gekennzeichnet, dass sich alle mechanisch-optischen Teile des Laser-Zusatzes in einem Gehäuse (34) befinden, das oberhalb der Mikroskopachsen des Beobachtungssystems angebracht ist und das auf der Benutzerseite eine Bedienungsplatte (34a) aufweist.

**Claims**

1. An ophthalmological combination instrument consisting of a stereoscopic microscope (31) and an auxiliary unit which is insertable instead of its main objective, characterized by the fact that as an auxiliary unit the combination of first laser means and second laser means is provided, said first laser means (9) having means for emitting pulsewise invisible radiation providing a plurality of individual pulses each having a duration of about 5 n sec, the individual pulses being time spaced from each other by about 10 µ sec, an energy of about 15 mJ being produced in one pumping operation, said radiation emitted by said first laser means forming a first beam, said second laser means (10) having means for emitting continuous visible radiation to form a second beam which, in cross section, consists of two individual beams (19, 19') which are alternatively to be switched on or off and which have a light-free zone between them, and means for directing said first beam and said second beam along a common axis (18) toward an object to be observed or treated.

2. The invention defined in claim 1, wherein said combination instrument includes an observation ray path (7) along which conversion filter means and laser protection filter means (28) interposed in said observation ray path.

3. The invention defined in claim 1, wherein said two individual beams (19, 19') of said second beam each have a cross section in the shape of a segment of a circle, the radius of said circle being larger than the radius of the circle of said first beam.

4. The invention defined in claim 3, further comprising means for alternately blocking and unblocking said two individual beams (19, 19') of said second beam.

5. The invention defined in claim 4, wherein said means for blocking and unblocking comprises a bar diaphragm (21) moved back and forth over the cross section of said second beam.

6. The invention defined in claim 5, wherein said bar diaphragm (21) consists of a spring wire and is shifted to and fro by a motor driven eccentric member (23).

7. The invention defined in claim 1, wherein said combination instrument has a microscope viewing system with viewing axes, further comprising a housing (34) arranged at least mainly above said viewing axes, said housing (34) containing substantially all mechanical-optical parts of said first laser means and said second laser means and being provided with a operating control plate (34a) on the side toward the user.

**Revendications**

1. Appareil ophtalmologique combiné, constitué d'un microscope d'observation stéréoscopique (31) et d'un appareil auxiliaire pouvant être mis à la place de son objectif principal, caractérisé en ce que l'appareil auxiliaire est formé par la combinaison d'un système laser, comprenant un laser (9) à émission pulsée dans le domaine invisible, qui génère, dans un cycle de pompage, plusieurs impulsions séparées d'une durée d'environ 5 ns chacune et avec un intervalle d'environ 10 µs entre les impulsions et une énergie d'environ 15 à 20 mJ, ainsi qu'un laser (10) émettant en continu dans le domaine visible, dont le faisceau est formé, en section droite, de deux faisceaux partiels (19, 19') qui peuvent être enenclenchés et coupés alternativement et entre lesquels se trouve une zone exempte de lumière, le rayon central (9'a) du rayonnement laser visible et le rayon central du rayonnement laser invisible étant situés sur un axe (18).

2. Appareil selon la revendication 1, caractérisé en ce que les faisceaux partiels (19, 19') possèdent, en section droite, la forme de segments d'un cercle dont le rayon est plus grand que le rayon du cercle délimitant la section droite du faisceau laser invisible (9').

3. Appareil selon la revendication 1, caractérisé en ce que des filtres de conversion et des filtres de protection (28) contre le rayonnement laser sont disposés dans le faisceau d'observation (7).

4. Appareil selon la revendication 2, caractérisé en ce qu'un montage électrique est prévu pour l'enclenchement et la coupure alternatifs du rayonnement laser visible (10').

5. Appareil selon la revendication 4, caractérisé en ce que l'enclenchement et la coupure des faisceaux partiels (19, 19') sont produits par un diaphragme tige (21) qui est animé d'un mouvement de va-et-vient sur la section droite du faisceau.

6. Appareil selon la revendication 5, caractérisé en ce que le diaphragme tige (21) est formé d'un fil à ressort qui est animé d'un mouvement de va-et-vient par un excentrique (23) entraîné en rotation par un moteur.

7. Appareil selon la revendication 1, caractérisé en ce que toutes les parties mécano-optiques de l'appareil auxiliaire à laser sont logées dans un boîtier (34) qui est disposé au-dessus des axes du microscope du système d'observation et qui présente une plaque de commande (34a) du côté de l'utilisateur.

Fig.1

## Fig. 2a

## Fig. 2b

Fig.3

# Fig.4a

# Fig.4b

# Fig.5